# FASCICULE DE BREVET EUROPEEN

(11) **EP 0 709 374 B1**
(45) Date de publication et mention de la délivrance du brevet: **18.06.1997**
(21) Numéro de dépôt: 95402267.9
(22) Date de dépôt: 10.10.1995
(51) Int. Cl.: C07D 209/08

(54) **Procédé de préparation de composés indoliques à partir d'indolines N-protégées**
Verfahren zur Herstellung von Indolderivaten ausgehend von N-geschützten Indolinderivaten
Process for the preparation of indol derivatives starting from N-protected indolin compounds

(30) Priorité: 27.10.1994 FR 9412892
(43) Date de publication de la demande: 01.05.1996
(73) Titulaire: L'OREAL, 75008 Paris (FR)
(72) Inventeur: Vandenbossche, Jean-Jacques, F-93270 Sevran (FR); Borowiak, Hervé, F-93270 Sevran (FR); Lagrange, Alain, F-77450 Coupvray (FR)
(74) Mandataire: Andral, Christophe André Louis

(56) Documents cités:
- EP-A- 0 604 278
- SYNTHESIS, STUTTGART DE, pages 778-781, TOSHIKO KIGUCHI ET AL. 'One-pot synthesis of indoles ...'
- TERAHEDRON LETTERS, vol. 33, no. 21, pages 2955-2956, JOGINDER S. BAJWA 'One-pot transformation of benzyl carbamates into t-butyl carbamates'
- SYNTHESIS, STUTTGART DE, pages 685-687, ALAN E. JACKSON ET AL. 'Rapid, selective removal of benzyloxycarbonyl groups ...'
- JOURNAL OF ORGANIC CHEMISTRY, vol. 43, no. 21, pages 4194-4196,
- Kocienski, Philip J.; Protecting Groups, Georg Thieme Verlag Stuttgart. New York (1994)

## Description

La présente invention concerne un nouveau procédé de préparation de composés indoliques à partir d'indolines N-protégées.

L'indole et ses dérivés sont des matières premières largement utilisées dans l'industrie des colorants, des cosmétiques, des produits pharmaceutiques ou alimentaires. L'indole est, en particulier, une des matières premières utilisables pour la synthèse du tryptophane qui est un acide aminé essentiel sur le plan nutritionnel.

La synthèse des indoles se fait en général en plusieurs étapes, de façon longue et coûteuse. De plus, cette synthèse nécessite des étapes d'oxydation poussées, à des températures de réaction élevées, souvent supérieures à 140 °C, entraînant des risques de manipulation.

Ainsi, l'article de KAMETANI et al., J.C.S. Perkin I, 290-294, 1981, décrit la synthèse de dérivés indoliques en deux étapes, à partir d'indolines N-protégées que l'on oxyde puis que l'on déprotège afin d'obtenir l'indole correspondant.

D'autre part, l'article de KIGUCHI et al., SYNTHESIS, 778-781, 1989, décrit la préparation de composés indoliques en une seule étape, par débenzylation et oxydation simultanées de 1-benzyl 2,3-dihydroindolines, en présence d'un catalyseur en milieu basique. Ce procédé présente certes l'avantage de ne comporter qu'une seule étape, mais cet article mentionne que de 3 à 15 % des indoles obtenus sont N-protégés par le groupement benzyle, ce qui n'est pas satisfaisant sur un plan industriel.

C'est en cherchant à remédier à ces problèmes que la demanderesse a découvert le procédé objet de l'invention.

La présente invention a donc pour objet un nouveau procédé de préparation de composés indoliques en une seule étape, qui est caractérisé par le fait qu'il consiste à mettre en contact, en présence d'un catalyseur d'hydrogénation, une indoline N-protégée de formule (I) suivante : 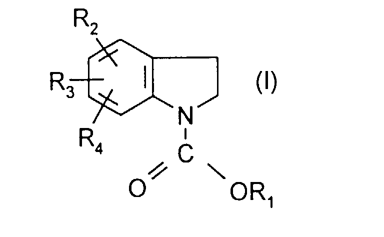 dans laquelle :
R₁ représente un radical alcényle linéaire ou ramifié en C₂-C₁₀ ou un groupement benzyle,
R₂, R₃ et R₄, identiques ou différents, représentent un atome d'hydrogène, un atome d'halogène tel que le brome, le chlore, le fluor ou l'iode, un radical alkyle en C₁-C₇, aryle, hydroxyle, alcoxy en C₁-C₇, aryloxy, acyloxy, formyle, aroyle, hydroxyméthyle, aryl-hydroxyméthyle, carboxy, alcoxy(C₁-C₇)carbonyle, carbamoyle, amino, monoalkyle(C₁-C₇)amino, dialkyle(C₁-C₇)amino, alcoxy(C₁-C₇)carbonylamino, acylamino, N-alkyl(C₁-C₇)acylamino, dialkyl(C₁-C₇)formamidino, ou dialcoxy(C₁-C₇) méthyle,
avec de l'hydrogène ou un donneur d'hydrogène, ladite mise en contact se faisant sous agitation, en milieu solvant, à un pH inférieur ou égal à 7 et à une température comprise entre la température ambiante et celle du reflux du milieu réactionnel, ce par quoi l'on obtient un composé indolique de formule (II) suivante : 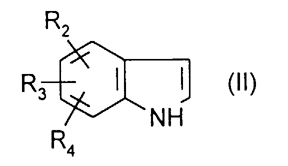 dans laquelle :
R₂, R₃ et R₄ ont les mêmes significations que dans la formule (I).

Selon ce procédé, il ne se forme pas de composé indolique N-protégé, ce qui présente un intérêt tant du point de vue de la pureté du produit final obtenu que du rendement de la réaction en ce produit.

Dans les formules (I) et (II) ci-dessus, le terme aryle désigne plus particulièrement un groupe phényle ou un groupe phényle portant un ou plusieurs substituants choisis parmi un atome d'halogène, un radical trifluorométhyle, un radical alkyle en C₁-C₇, alcoxy en C₁-C₇, un groupement nitro, amino, alkyl(C₁-C₇)amino et dialkyl(C₁-C₇)amino.

Le radical acyle désigne de préférence un radical alcanoyle dérivé d'un acide carboxylique aliphatique ayant de 1 à 7 atomes de carbone tels que par exemple les groupes formyle, acétyle, propionyle, etc ; ou un groupe aroyle dérivé d'un acide carboxylique aromatique tel que le groupe benzoyle.

Les indolines de départ de formule (I) sont des produits connus qui peuvent par exemple être préparés selon le procédé décrit dans la demande de brevet français FR-A-2 699 532.

Parmi les indolines de formule (I), on peut citer :
- la 5,6-dihydroxy 1-N-benzyloxycarbonylindoline,
- la 5,6-dihydroxy 1-N-allyloxycarbonylindoline,
- la 6-hydroxy 1-N-benzyloxycarbonylindoline,
- la 4-hydroxy 5-méthoxy 1-N-benzyloxycarbonylindoline,
- la 6-hydroxy 7-méthoxy 1-N-benzyloxycarbonylindoline,
- la 5-méthoxy 6-hydroxy 1-N-benzyloxycarbonylindoline,
- la 1-N-benzyloxycarbonylindoline.

Parmi les composés indoliques de formule (Il) que l'on peut obtenir, on peut citer :
- le 5,6-dihydroxyindole,
- le 6-hydroxyindole,
- le 4-hydroxy 5-méthoxyindole,
- le 6-hydroxy 7-méthoxyindole,
- le 5-méthoxy 6-hydroxyindole,
- l'indole.

Selon un mode de réalisation préféré du procédé selon l'invention, le pH du milieu solvant est compris entre 5 et 7 et encore plus préférentiellement est compris entre 6,5 et 7.

L'hydrogène utilisé selon le procédé de l'invention est de préférence de l'hydrogène moléculaire sous faible pression.

Parmi les donneurs d'hydrogène, on peut citer les donneurs d'hydrogène oléfiniques tels que par exemple le cyclohexène, les cyclohexènes substitués, le 1,3-cyclohexadiène et le 1,4-cyclohexadiène.

Selon le procédé de l'invention, on peut employer tout catalyseur d'hydrogénation approprié, par exemple un métal choisi dans le groupe constitué par le chrome, le molybdène, le tungstène, le platine, le palladium, le rhodium, le cobalt, le nickel et le ruthénium, leurs oxydes, et les combinaisons de ces substances, telles que par exemple un mélange d'oxyde de cobalt et de molybdène y compris le molybdate de cobalt.

Les catalyseurs d'hydrogénation préférés sont le palladium ou le nickel de Raney, de même que d'autres métaux du groupe du platine. De façon connue, le catalyseur peut être déposé sur un support inerte de pH neutre de façon à ne pas influencer le pH du milieu solvant réactionnel. Parmi ces supports inertes, on peut citer par exemple le charbon de bois neutre, le charbon neutre, l'alumine neutre, les zéolithes, les argiles, etc. On utilise de préférence du charbon neutre.

Les catalyseurs d'hydrogénation sont en général présents en une quantité comprise entre 0,2 et 5 % en poids d'équivalent métal par rapport au poids du composé de formule (I) à faire réagir.

D'une manière avantageuse, le solvant utilisé est un solvant inerte comme par exemple un alcool en C₁-C₄ tel que le méthanol ou l'éthanol, un hydrocarbure tel que le benzène ou le toluène, l'acétate d'éthyle, le diméthyle formamide ou leurs mélanges. De façon préférentielle, le solvant utilisé est l'éthanol ou l'acétate d'éthyle.

Lorsque la réaction est terminée, les produits attendus, tels que par exemple l'indole, peuvent, le cas échéant, être récupérés par des méthodes bien connues de l'état de la technique telles que la cristallisation, la distillation ou l'entraînement à la vapeur.

Les produits obtenus selon le procédé de l'invention, c'est à dire les composés indoliques de formule (II) dont l'indole, peuvent être utilisés à de nombreuses fins, par exemple comme composés intermédiaires dans la préparation d'acides aminés, d'alcaloïdes de tryptamines, etc..., ou encore comme produits finis dans tout type d'industrie, chimique, cosmétique, alimentaire, pharmaceutique, ou autre.

Les exemples de préparation qui suivent sont destinés à illustrer l'invention.

### EXEMPLES

### EXEMPLE 1: Synthèse du 5,6-dihydroxyindole

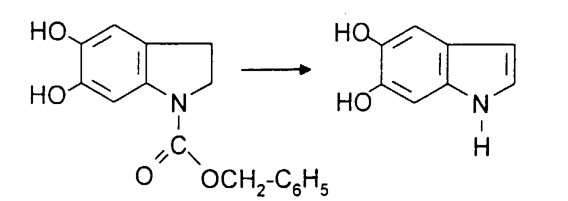

On a mélangé 11,4 g d'ester benzylique de l'acide 5,6-dihydroxy-2,3-dihydroindole 1-carboxylique, 60 ml d'éthanol, 5,7 g de palladium sur charbon à 10 %, 5,7 ml d'eau et 23 ml de cyclohexène et on a porté l'ensemble au reflux pendant une heure.
On a filtré le catalyseur à température ambiante, puis a on évaporé à sec. Le résidu beige a été repris dans l'éther isopropylique à chaud en présence de charbon, puis on a filtré à chaud et évaporé la phase organique. On a obtenu 4,5 g d'un solide beige clair (produit attendu) dont le point de fusion est de 144°C et dont l'analyse élémentaire pour C₈H₇NO₂ est la suivante :

| | **%** | **C** | **H** | **N** | **O** |
|---|---|---|---|---|---|
| **Calculée** | | 64,42 | 4,73 | 9,39 | 21,45 |
| **Trouvée** | | 64,20 | 4,91 | 9,41 | 21,44 |

### EXEMPLE 2 : Synthèse du 6-hydroxyindole

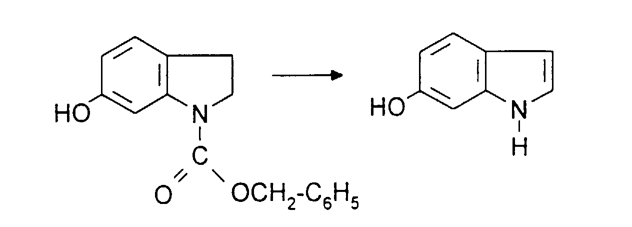

On a utilisé le procédé décrit à l'exemple 1 ci-dessus, en partant de 5,4 g de l'ester benzylique de l'acide 6-hydroxy-2,3-dihydroindole 1-carboxylique.

On a obtenu 2 g de cristaux blancs (produit attendu) dont le point de fusion est de 130°C et dont l'analyse élémentaire pour C₈H₇NO est :

| | **%** | **C** | **H** | **N** | **O** |
|---|---|---|---|---|---|
| **Calculée** | | 72,17 | 5,30 | 10,52 | 12,02 |
| **Trouvée** | | 72,18 | 5,31 | 10,49 | 11,99 |

## Revendications

1. Procédé de préparation de composés indoliques en une seule étape, caractérisé par le fait qu'il consiste à mettre en contact, en présence d'un catalyseur d'hydrogénation, une indoline N-protégée de formule (I) suivante : 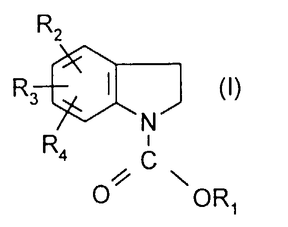 dans laquelle :
R₁ représente un radical alcényle linéaire ou ramifié en C₂-C₁₀ ou un groupement benzyle,
R₂, R₃ et R₄, identiques ou différents, représentent un atome d'hydrogène, un atome d'halogène tel que le brome, le chlore, le fluor ou l'iode, un radical alkyle en C₁-C₇, aryle, hydroxyle, alcoxy en C₁-C₇, aryloxy, acyloxy, formyle, aroyle, hyd roxyméthyle, aryl-hydroxyméthyle, carboxy, alcoxy(C₁-C₇)carbonyle, carbamoyle, amino, monoalkyle(C₁-C₇)amino, dialkyle(C₁-C₇)amino, alcoxy(C₁-C₇)carbonylamino, acylamino, N-alkyl(C₁-C₇)acylamino, dialkyl(C₁-C₇)formamidino, ou dialcoxy(C₁-C₇) méthyle,
avec de l'hydrogène ou un donneur d'hydrogène, ladite mise en contact se faisant sous agitation, en milieu solvant, à un pH inférieur ou égal à 7 et à une température comprise entre la température ambiante et celle du reflux du milieu réactionnel, ce par quoi l'on obtient un composé indolique de formule (II) suivante : 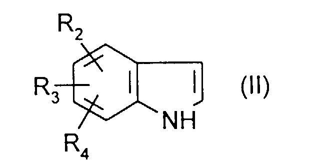 dans laquelle :
R₂, R₃ et R₄ ont les mêmes significations que dans la formule (I).

2. Procédé selon la revendication 1, caractérisé par le fait que dans les formules (I) et (Il) le terme aryle désigne un groupe phényle ou un groupe phényle portant un ou plusieurs substituants choisis parmi un atome d'halogène, un radical trifluorométhyle, un radical alkyle en C₁-C₇, alcoxy en C₁-C₇, un groupement nitro, amino, alkyle(C₁-C₇)amino et dialkyle(C₁-C₇)amino, et que le radical acyle désigne un radical alcanoyle dérivé d'un acide carboxylique aliphatique ayant de 1 à 7 carbone ou un groupe aroyle dérivé d'un acide carboxylique aromatique.

3. Procédé selon l'une quelconque des revendications 1 ou 2, caractérisé par le fait que les indolines de formule (I) sont choisies parmi la 5,6-dihydroxy 1-N-benzyloxycarbonylindoline, la 5,6-dihydroxy 1-N-allyloxycarbonylindoline, la 6-hydroxy 1-N-benzyloxycarbonylindoline, la 4-hydroxy 5-méthoxy 1-N-benzyloxycarbonylindoline, la 6-hydroxy 7-méthoxy 1-N-benzyloxycarbonylindoline, la 5-méthoxy 6-hydroxy 1-N-benzyloxycarbonylindoline, la 1-N-benzyloxycarbonylindoline.

4. Procédé selon l'une quelconque des revendications 1 à 3, pour la préparation du 5,6-dihydroxyindole, du 6-hydroxyindole, du 4-hydroxy 5-méthoxyindole, du 6-hydroxy 7-méthoxyindole, du 5-méthoxy 6-hydroxyindole ou de l'indole.

5. Procédé selon l'une quelconque des revendications 1 à 4, caractérisé par le fait que le pH du milieu solvant est compris entre 5 et 7.

6. Procédé selon l'une quelconque des revendications 1 à 5, caractérisé par le fait que l'hydrogène est de l'hydrogène moléculaire sous faible pression.

7. Procédé selon l'une quelconque des revendications 1 à 5, caractérisé par le fait que les donneurs d'hydrogène sont choisis parmi les donneurs d'hydrogène oléfiniques tels que le cyclohexène, les cyclohexènes substitués, le 1,3-cyclohexadiène et le 1,4-cyclohexadiène.

8. Procédé selon l'une quelconque des revendications 1 à 7, caractérisé par le fait que le catalyseur d'hydrogénation est un métal choisi dans le groupe constitué par le chrome, le molybdène, le tungstène, le platine, le palladium, le rhodium, le cobalt, le nickel et le ruthénium, leurs oxydes, et les combinaisons de ces substances, telles qu'un mélange d'oxyde de cobalt et de molybdène comme le molybdate de cobalt.

9. Procédé selon la revendication 8, caractérisé par le fait que le catalyseur d'hydrogénation est le palladium ou le nickel de Raney ou un autre métal du groupe du platine.

10. Procédé selon la revendication 8 ou 9, caractérisé par le fait que le catalyseur d'hydrogénation est placé sur du charbon de bois neutre, du charbon neutre, de l'alumine neutre, des zéolithes ou des argiles.

11. Procédé selon l'une quelconque des revendications 1 à 10, caractérisé par le fait que le solvant utilisé est un solvant inerte choisi parmi les alcools en C₁-C₄, les hydrocarbures, l'acétate d'éthyle et le diméthyle formamide.

12. Procédé selon la revendication 11, caractérisé par le fait que le solvant est choisi parmi l'éthanol et l'acétate d'éthyle.

## Patentansprüche

1. Verfahren zur Herstellung von Indolverbindungen in einem einzigen Schritt, dadurch gekennzeichnet, daß es darin besteht, eine N-geschützte Indolinverbindung der folgenden Formel (I) 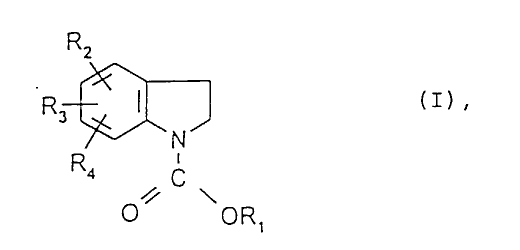 worin bedeuten:
R₁ eine geradkettige oder verzweigte C₂₋₁₀-Alkenylgruppe oder eine Benzylgruppe,
R₂, R₃ und R₄, die gleich oder voneinander verschieden sind, Wasserstoff, Halogen, wie Brom, Chlor, Fluor oder Jod, C₁₋₇-Alkyl, Aryl, Hydroxy, C₁₋₇-Alkoxy, Aryloxy, Acyloxy, Formyl, Aroyl, Hydroxymethyl, Arylhydroxymethyl, Carboxy, C₁₋₇-Alkoxycarbonyl, Carbamoyl, Amino, C₁₋₇-Monoalkylamino, C₁₋₇-Dialkylamino, C₁₋₇-Alkoxycarbonylamino, Acylamino, C₁₋₇-N-Alkylacylamino, C₁₋₇-Dialkylformamidino oder C₁₋₇Dialkoxymethyl,
in Gegenwart eines Hydrierungskatalysators mit Wasserstoff oder einem Wasserstoff-Donator in Kontakt zu bringen, wobei das Inkontaktbringen unter Rühren in einem Lösungsmittelmedium bei einem pH-Wert von höchstens 7 und einer Temperatur im Bereich von Raumtemperatur und der Rückflußtemperatur des Reaktionsmediums durchgeführt wird, wodurch eine Indolverbindung der folgenden Formel (II) hergestellt wird: 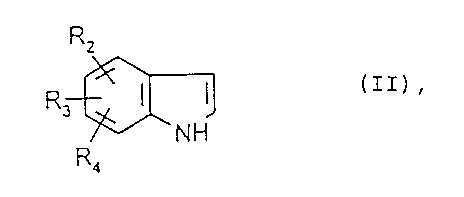 worin
R₂, R₃ und R₄ die für die Formel (I) angegebenen Bedeutungen aufweisen.

2. Verfahren nach Anspruch 1, dadurch gekennzeichnet, daß in den Formeln (I) und (II) Aryl eine Phenylgruppe oder eine Phenylgruppe, die einen oder mehrere Substituenten aufweist, die unter Halogen, Trifluormethyl, C₁₋₇-Alkyl, C₁₋₇-Alkoxy, Nitro, Amino, C₁₋₇-Alkylamino und C₁₋₇-Dialkylamino ausgewählt sind, und Acyl eine Alkanoylgruppe, die von einer aliphatischen Carbonsäure mit 1 bis 7 Kohlenstoffatomen abgeleitet ist, oder eine Aroylgruppe bedeutet, die von einer aromatischen Carbonsäure abgeleitet ist.

3. Verfahren nach einem der Ansprüche 1 oder 2, dadurch gekennzeichnet, daß die Indolinverbindungen der Formel (I) unter 5,6-Dihydroxy-1-N-benzyloxycarbonylindolin, 5,6-Dihydroxy-1-N-allyloxycarbonylindolin, 6-Hydroxy-1-N-benzyloxycarbonylindolin, 4-Hydroxy-5-methoxy-1-N-benzyloxycarbonylindolin, 6-Hydroxy-7-methoxy-1-N-benzyloxycarbonylindolin, 5-Methoxy-6-hydroxy-1-N-benzyloxycarbonylindolin und 1-N-Benzyloxycarbonylindolin ausgewählt sind.

4. Verfahren nach einem der Ansprüche 1 bis 3 zur Herstellung von 5,6-Dihydroxyindol, 6-Hydroxyindol, 4-Hydroxy-5-methoxyindol, 6-Hydroxy-7-methoxyindol, 5-Methoxy-6-hydroxyindol oder Indol.

5. Verfahren nach einem der Ansprüche 1 bis 4, dadurch gekennzeichnet, daß der pH-Wert des Lösungsmittelmediums im Bereich von 5 bis 7 liegt.

6. Verfahren nach einem der Ansprüche 1 bis 5, dadurch gekennzeichnet, daß der Wasserstoff molekularer Wasserstoff unter geringem Druck ist.

7. Verfahren nach einem der Ansprüche 1 bis 5, dadurch gekennzeichnet, daß die Wasserstoff-Donatoren unter den olefinischen Wasserstoff-Donatoren ausgewählt sind, wie Cyclohexen, substituierten Cyclohexenen, 1,3-Cyclohexadien und 1,4-Cyclohexadien.

8. Verfahren nach einem der Ansprüche 1 bis 7, dadurch gekennzeichnet, daß der Hydrierungskatalysator ein Metall ist, das unter Chrom, Molybdän, Wolfram, Platin, Palladium, Rhodium, Cobalt, Nickel und Ruthenium, deren Oxiden und Kombinationen dieser Subsanzen ausgewählt ist, wie einem Gemisch von Cobaltoxid und Molybdänoxid, wie beispielsweise Cobaltmolybdat.

9. Verfahren nach Anspruch 8, dadurch gekennzeichnet, daß der Hydrierungskatalysator Palladium oder Raney-Nickel oder ein weiteres Metall der Platingruppe ist.

10. Verfahren nach Anspruch 8 oder 9, dadurch gekennzeichnet, daß der Hydrierungskatalysator auf neutraler Holzkohle, neutraler Kohle, neutralem Aluminiumoxid, Zeolithen oder Tonen aufgebracht ist.

11. Verfahren nach einem der Ansprüche 1 bis 10, dadurch gekennzeichnet, daß das verwendete Lösungsmittel ein inertes Lösungsmittel ist, das unter den C₁₋₄-Alkoholen, Kohlenwasserstoffen, Ethylacetat und Dimethylformamid ausgewählt ist.

12. Verfahren nach Anspruch 11, dadurch gekennzeichnet, daß das Lösungsmittel unter Ethanol und Ethylacetat ausgewählt ist.

## Claims

1. Process for the preparation of indole compounds in a single step, characterized in that it consists in bringing together, in the presence of a hydrogenation catalyst, an N-protected indoline of formula (I) below: 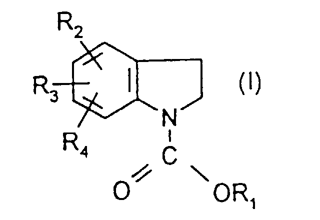 in which:
R₁ represents a linear or branched C₂-C₁₀ alkenyl radical or a benzyl group,
R₂, R₃ and R₄, which may be identical or different, represent a hydrogen atom, a halogen atom such as bromine, chlorine, fluorine or iodine, or a C₁-C₇ alkyl, aryl, hydroxyl, C₁-C₇ alkoxy, aryloxy, acyloxy, formyl, aroyl, hydroxymethyl, arylhydroxymethyl, carboxyl, alkoxy(C₁-C₇)carbonyl, carbamoyl, amino, monoalkyl(C₁-C₇)-amino, dialkyl(C₁-C₇)amino, alkoxy(C₁-C₇)carbonylamino, acylamino, N-alkyl(C₁-C₇)acylamino, dialkyl(C₁-C₇)formamidino or dialkoxy(C₁-C₇)methyl radical,
and hydrogen or a hydrogen donor, the said bringing together being carried out with stirring, in a solvent medium, at a pH below or equal to 7 and at a temperature of between room temperature and that of reflux of the reaction medium, by which means an indole compound is obtained, of formula (II) below: 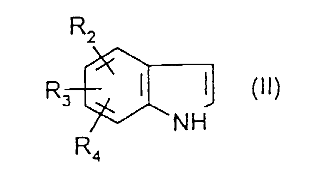 in which:
R₂, R₃ and R₄ have the same meanings as in formula (I).

2. Process according to Claim 1, characterized in that, in the formulae (I) and (II), the term aryl more particularly denotes a phenyl group or a phenyl group bearing one or more substituents chosen from a halogen atom, a trifluoromethyl radical, a C₁-C₇ alkyl or C₁-C₇ alkoxy radical and a nitro, amino, alkyl(C₁-C₇)amino or dialkyl(C₁-C₇)amino group, the acyl radical denotes an alkanoyl radical derived from an aliphatic carboxylic acid having from 1 to 7 carbon atoms or an aroyl group derived from an aromatic carboxylic acid.

3. Process according to either of Claims 1 and 2, characterized in that the indolines of formula (I) are chosen from 5,6-dihydroxy-1-N-benzyloxycarbonylindoline, 5,6-dihydroxy-1-N-allyloxycarbonylindoline, 6-hydroxy-1-N-benzyloxycarbonylindoline, 4-hydroxy-5-methoxy-1-N-benzyloxycarbonylindoline, 6-hydroxy-7-methoxy-1-N-benzyloxycarbonylindoline, 5-methoxy-6-hydroxy-1-N-benzyloxycarbonylindoline and 1-N-benzyloxycarbonylindoline.

4. Process according to any one of Claims 1 to 3, for the preparation of 5,6-dihydroxyindole, 6-hydroxyindole, 4-hydroxy-5-methoxyindole, 6-hydroxy-7-methoxyindole, 5-methoxy-6-hydroxyindole or indole.

5. Process according to any one of Claims 1 to 4, characterized in that the pH of the solvent medium is between 5 and 7.

6. Process according to any one of Claims 1 to 5, characterized in that the hydrogen is molecular hydrogen at low pressure.

7. Process according to any one of Claims 1 to 5, characterized in that the hydrogen donors are chosen from olefinic hydrogen donors such as cyclohexene, substituted cyclohexenes, 1,3-cyclohexadiene and 1,4-cyclohexadiene.

8. Process according to any one of Claims 1 to 7, characterized in that the hydrogenation catalyst is a metal chosen from the group consisting of chromium, molybdenum, tungsten, platinum, palladium, rhodium, cobalt, nickel and ruthenium, oxides thereof and combinations of these substances, such as a mixture of cobalt oxide and molybdenum oxide, including cobalt molybdate.

9. Process according to Claim 8, characterized in that the hydrogenation catalyst is palladium or Raney nickel or another metal from the platinum group.

10. Process according to Claim 8 or 9, characterized in that the hydrogenation catalyst is placed on neutral wood charcoal, neutral charcoal, neutral alumina, zeolites or clays.

11. Process according to any one of Claims 1 to 10, characterized in that the solvent used is an inert solvent chosen from C₁-C₄ alcohols, hydrocarbons, ethyl acetate and dimethylformamide.

12. Process according to Claim 11, characterized in that the solvent is chosen from ethanol and ethyl acetate.
